# EUROPEAN PATENT SPECIFICATION

(11) **EP 3 383 505 B1**
(45) Date of publication and mention of the grant of the patent: **11.11.2020**
(21) Application number: 15825770.9
(22) Date of filing: 31.12.2015
(51) Int. Cl.: A61Q 19/10, A61K 8/41, A61K 8/42, A61Q 5/02, A61Q 5/12, A61Q 15/00, A61Q 19/00, A61K 8/81, A61K 8/04, A61Q 17/00

(54) **PERSONAL CARE COMPOSITIONS**
KÖRPERPFLEGEZUSAMMENSETZUNGEN
COMPOSITIONS DE SOIN PERSONNEL

(43) Date of publication of application: 10.10.2018
(73) Proprietor: Colgate-Palmolive Company, New York, NY 10022 (US)
(72) Inventor: HARDY, Eugene, Old Bridge New Jersey 08857 (US); KUGLER, Alison, Morganville New Jersey 07751 (US)
(74) Representative: Wichmann, Hendrik
(86) International application number: PCT/US2015/068262
(87) International publication number: WO 2017/116462

(56) References cited:
- EP-A1- 2 221 044
- WO-A1-2015/047260
- WO-A1-2015/073117
- WO-A2-2009/156248
- WO-A2-2014/099164
- FR-A1- 2 920 974
- US-A1- 2010 278 906
- US-A1- 2011 207 832
- ALLEN PARK ET AL: "Carbopol TM Aqua CC Polymer: The Premier Cationic Compatible Rheology Modifier for Low pH Formulations", INTERNET CITATION, 1 January 2006 (2006-01-01), pages 1-8, XP002744420, Retrieved from the Internet: URL:http://www.lubrizol.com/PersonalCare/P roducts/Carbopol/CarbopolAquaCC.html [retrieved on 2016-06-29]

## Description

### BACKGROUND

Cationic surfactants can provide antibacterial properties to a cleansing composition. However, when cationic surfactants are mixed with nonionic surfactants, the resulting composition may not have rheological properties desirable for a personal care composition. In many applications, for example, in liquid soap, creams or lotions, it is desirable for the product to be flowable, possess antibacterial qualities and pleasant sensory qualities for the consumer, and easily form a foam when agitated. In systems comprising a mixture of cationic and nonionic surfactants, there is generally a need to use thickeners, viscosity enhancing agents or structurants to increase the viscosity to a level that is expected by consumers for cleansing compositions. However, surfactant systems have become increasingly complex in composition. In many cationic-based skin cleansing systems there is a greater challenge in finding appropriate structurants that can provide the necessary rheology profile to suspend particles and active agents to deliver sensory and/or cosmetic benefits and other performance properties while maintaining acceptable long-term product stability in low pH surfactant systems at standard aging conditions.

Thus, there is a need for a cosmetic or antibacterial personal care cleansing composition comprising a structurant capable of suspending beads and other particles in a low pH liquid cleansing composition containing both cationic and nonionic surfactants. It is also desirable that the composition provides a balance of acceptable physical and performance properties, such as flowability, antibacterial and foaming profiles, as well as pleasant sensory qualities for the consumer. Embodiments of the present invention are designed to meet these needs.
Allen Park et al. disclose in "Carbopol® Aqua CC Polymer: The Premier Cationic Compatible Rheology Modifier for Low pH Formulations" in Cosmetic Science Technology 2006 a composition comprising deionized water, polyquaternium-10, polyacrylate-1 crosspolymer (20) in an amount of 6.25 wt%, cocamide MEA and other ingredients.

### BRIEF SUMMARY

Provided herein are personal care cleansing compositions comprising a structurant capable of suspending beads and other particles in a low pH liquid cleansing composition that includes both cationic and nonionic surfactants as laid out in the claims. Also provided are compositions that provide a balance of acceptable physical and performance properties, such as flowability, antibacterial and foaming profiles, as well as pleasant sensory qualities for the consumer.

It has been found that the use of a polyacrylate, such as, for example, polyacrylate-1 crosspolymer, in a personal cleansing system comprising a combination of compatible cationic and nonionic surfactants provides a balance of desirable formulation properties. It has been found that in concentrations above 1 wt % a polyacrylate-1 crosspolymer unexpectedly increases the foam profile of a cationic and nonionic surfactant system and provides the system with the ability to suspend particles for prolonged periods of time.

In a first embodiment, provided is a personal care composition comprising a cationic surfactant; a nonionic surfactant comprising at least one surfactant chosen from an amine oxide and a fatty acid amide; and a structuring agent comprising polyacrylate-1 crosspolymer in an amount of 3 wt to 7 wt %, by weight of the composition, and wherein the cationic surfactant is a quaternary ammonium alkyl salt.

In a second exemplary embodiment, provided is a method of cleansing skin comprising the steps of providing a personal care composition comprising a cationic surfactant, a nonionic surfactant comprising at least one surfactant chosen from an amine oxide and a fatty acid amide, and a structuring agent comprising polyacrylate-1 crosspolymer in an amount of 3 wt % of the compositionto 7 wt % and wherein the cationic surfactant is a quaternary ammonium alkyl salt; and applying the composition to the skin to provide a cleansing effect.

### DETAILED DESCRIPTION

Unless otherwise specified, all percentages and amounts expressed herein and elsewhere in the specification should be understood to refer to percentages by weight. Also, the term "about," when used in reference to a range of values, should be understood to refer to either value in the range, or to both values in the range.

The present disclosure provides personal care compositions comprising a polyacrylate polymer added to a cationic and nonionic surfactant system having a low pH. In various embodiments, the polyacrylate polymer includes, or consists of, polyacrylate-1 crosspolymer. It has been found that concentrations between about 1.5 wt % and about 7 wt % of a polyacrylate-1 crosspolymer unexpectedly increases the foam profile of a cationic and nonionic surfactant system and provides the system with the ability to suspend particles for prolonged periods of time.

The present invention provides a personal care composition (Composition 1) comprising:
a cationic surfactant;
a nonionic surfactant system comprising at least one surfactant chosen from an amine oxide surfactant and a fatty acid amide surfactant; and
a structuring agent comprising polyacrylate-1 crosspolymer in an amount of 3 wt % to 7 wt % by weight of the composition, and wherein the cationic surfactant is a quaternary ammonium alkyl salt.

The present disclosure provides additional exemplary embodiments, including:
A Composition as defined above, wherein the polyacrylate-1 crosspolymer is present in an amount of 5%, 4 wt % or 3 wt % by weight of the composition.
The cationic surfactant can comprise an alkyltrimethylammonium salt.
The cationic surfactant can comprise cetyltrimethylammonium chloride.
The composition can further comprise a cationic antibacterial agent.
The composition can further comprise a cationic antibacterial agent selected from benzalkonium chloride, benzethonium chloride, and combinations thereof.
The nonionic surfactant system can comprise laurylamidopropyl dimethylamine oxide, myristylamidopropyl dimethylamine oxide, cocomonoethanolamide, or combinations thereof.
The composition can not contain an anionic surfactant.
The composition can be in the form of a liquid hand soap, shampoo, conditioner, liquid face soap, dish soap, antiperspirant, deodorant, body wash, dermal lotion, dermal cream, dermal conditioner, or liquid detergent.
The cationic surfactant can be present in an amount of about 0.1 wt % to about 10 wt% of the composition.
The cationic surfactant can be present in an amount of about 0.1 wt % to about 5 wt %, about 0.5 wt % to about 10 wt %, about 0.5 wt % to about 5 wt %, about 1 wt % to about 5 wt %, or about 2 wt % to about 4 wt % of the composition.
The nonionic surfactant system is present in an amount of about 0.1 wt % to about 6.0 wt % of the composition.
The nonionic surfactant system can be present in an amount of about 0.5 wt % to about 10 wt %, about 0.5 wt % to about 5 wt %, or about 0.5 wt % to about 3 wt % of the composition.
The composition can have a shear storage modulus to shear loss modulus ratio from about 1:1 to about 7:1.
The composition can have a shear storage modulus to shear loss modulus ratio from about 2:1 to about 4:1.
The composition can have a ratio of cationic surfactant to total nonionic surfactant of from 0.8:1 to 1:0.8.
The composition can further comprise one or more ingredients selected from among:
(a) Humectants (e.g., glycerin, sorbitol, propylene glycol),
(b) Fatty acids (e.g., caproic acid, caprylic acid, capric acid, lauric acid, myristic acid, palmitic acid, palmitoleic acid, stearic acid, oleic acid, linolenic acid, linoleic acid, arachidic acid, arachidonic acid),
(c) Fatty alcohols (e.g., cetearyl alcohol, cetyl alcohol, stearyl alcohol, oleyl alcohol),
(d) Esters of fatty acids (e.g., esters of caproic acid, caprylic acid, capric acid, lauric acid, myristic acid, palmitic acid, palmitoleic acid, stearic acid, oleic acid, linolenic acid, linoleic acid, arachidic acid, arachidonic acid, with alcohols such as glycerol, propylene glycol, sorbitan, isopropyl alcohol, caproic alcohol, capryl alcohol, capric alcohol, lauryl alcohol, myristyl alcohol, cetearyl alcohol, cetyl alcohol, palmoleyl alcohol, stearyl alcohol, oleyl alcohol, linoyl alcohol, linolenyl alcohol, arachidyl alcohol, arachidonyl alcohol) such as isopropyl myristate, capryl stearate, isopropyl olivate, cetearyl olivate, cetearyl oleate, glyceryl caprylate, glyceryl stearate citrate, and sorbitan olivate), natural and synthetic triglycerides (e.g., di- or tri-glycerides of fatty acids, such as glyceryl caprate or caprylic/capric triglyceride),
(e) Natural fats and oils (e.g., vegetable oil, coconut oil, sesame oil, avocado oil, corn oil, castor oil, shea butter, cocoa butter, soybean oil, sunflower oil, safflower oil, olive oil and tallow),
(f) Waxes (e.g., cetearyl wax, beeswax, carnauba wax, lanolin wax, candelilla wax, and paraffin wax),
(g) Thickeners (e.g., silicas, xanthan gum, guar gum, agar, alginates, carrageenan, gellan gum, pectins, and modified cellulose polymers, such as hydroxycellulose, hydroxypropyl cellulose, hydroxyethyl cellulose, hydroxybutyl cellulose, hydropropyl methylcellulose, hydroxyethyl propyl cellulose),
(h) Emulsifiers (e.g. polyethylene glycol esters, fatty alcohol polyglycol ethers, fatty acid polyglycol ethers, polyglycerol fatty acid esters, sorbitol, sorbitan, and mono- and di-fatty acid esters of sorbitan),
(i) Sunscreen actives (e.g., titanium dioxide, zinc oxide, and UV absorption inhibitors, such as octyl methoxy cinnamate, benzophenone-3, and methylene bis-benzotriazolyl tetramethyl butyl phenol),
(j) Vitamins (e.g., vitamin A, vitamin E, esters of vitamin A or vitamin E, such as vitamin E acetate and retinyl palmitate).
The composition can be a cream, lotion or gel for the skin (e.g., face, hands, feet, etc.).
The composition can further comprising inorganic salts, brighteners, perfumes, colorants, sequestering agents, opacifiers, pearlizers, chelating agents (e.g., EDTA), or any combination thereof.
The composition can be a cosmetic product, cosmetic-removal product, deodorant or antiperspirant product, hair care product, shaving product (e.g., creams, gels and foams), sun bathing product (e.g., sunscreen compositions and tanning compositions), insect repellent product, skin care product or personal cleansing product (e.g., liquid soaps, foams, gels, and lotions).
The composition can further comprise natural biological extracts, such as essential oils or fragrances (e.g., Amyris oil, cedarwood oil, cocoa absolute, copaiba balsam, menthe oil pays, myrrh resin, patchouli oil, vanillin, vetiver oil, Aloe extract, lemon extract, orange extract, mandarin extract, and oil or extract of anise, clove, basil, aniseed, cinnamon, geranium, rose, mint, lavender, thyme, rosemary, citronella, cypress, eucalyptus, peppermint, and sandalwood).
The composition can further comprise water, e.g., from 5-90% water by weight of the composition, for example, 10%-80%, 15%-80%, 20%-80%, 25%-80%, 25%-75%, 30%-75%, 30%-80%, 40%-80%, 40%-70%, 50%-75%, 50%-70%, 50%-65%, or 60%-70%, or 65-70%, or about 65%, or about 66%, or about 67%, or about 68%.
The pH of the composition can be from 1-8, for example, from 3.0-7.0, from 4.0-6.0, from 4.8-5.4, or about 5.0.
The composition can further comprise one or more types of suspended particles.
The particles can be beads.
The beads can be selected from alginate beads, agar beads, carrageenan beads, and combinations thereof.

The present personal care compositions include a high load of cationic and nonionic surfactants, and a structuring agent comprising polyacrylate-1 crosspolymer. The compositions are particularly compatible with cationic antibacterial agents, such as for example benzalkonium chloride.

The novel compositions of the present disclosure are capable of suspending particulate material. For a composition to suspend particles, the shear storage modulus (G') of the composition must be greater than the shear loss modulus (G") of the composition. That is, the ratio of G':G" must be greater than 1:1. In some embodiments of the present compositions, the ratio of the shear storage modulus (G') to the shear loss modulus (G") of the composition is between about 1:1 to about 7:1, or about 2:1 to about 4:1. In some embodiments, the ratio is about 1.5:1, about 1.6:1, about 1.7:1, about 1.8:1, about 1.9:1, about 2.0:1, about 2.1:1, about 2.2:1, about 2.3:1, about 2.4:1, about 2.5:1, about 2.6:1, about 2.7:1, about 2.8:1, about 2.9:1, about 3.0:1, about 3.1:1, about 3.2:1, about 3.3:1, about 3.4:1, about 3.5:1, about 3.6:1, about 3.7:1, about 3.8:1; about 3.9:1; or about 4.0:1.

The present compositions include at least one a cationic surfactant. The cationic surfactant can be any cationic surfactant suitable for use in personal care compositions. In the invention, the cationic surfactant includes a quaternary ammonium alkyl salt, for example an alkyltrimethylammonium salt. In some embodiments, the salt can be a halide, such as chloride or bromide, or a methosulfate. In some embodiments, the alkyl portion of the quaternary ammonium alkyl salt can be a C₈-C₂₄ alkyl or a C₁₄-C₁₈ alkyl. In certain embodiments, the cationic surfactant is cetyltrimethylammonium chloride. In some embodiments, the composition includes a mixture of one or more cationic surfactants. In certain embodiments, the cationic surfactant is present in an amount of about 0.1 to about 20 wt % of the composition; or from about 0.1 to about 10, about 0.1 to about 5, about 0.5 to about 10, about 0.5 to about 5, about 1 to about 5, or about 2 to about 4 wt % of the composition.

The present compositions include at least one non-ionic surfactant, which together are referred to herein as a non-ionic surfactant system. Nonionic surfactants that can be used in the compositions can broadly be defined as compounds produced by the condensation of alkylene oxide groups (hydrophilic in nature) with an organic hydrophobic compound which may be aliphatic or alkyl-aromatic in nature. Examples of suitable nonionic surfactants include poloxamers (sold under trade name PLURONIC®), polyoxyethylene, polyoxyethylene sorbitan esters (sold under trade name TWEENS®), Polyoxyl 40 hydrogenated castor oil, fatty alcohol ethoxylates, polyethylene oxide condensates of alkyl phenols, products derived from the condensation of ethylene oxide with the reaction product of propylene oxide and ethylene diamine, ethylene oxide condensates of aliphatic alcohols, alkyl polyglycosides (for example, fatty alcohol ethers of polyglycosides, such as fatty alcohol ethers of polyglucosides, e.g., decyl, lauryl, capryl, caprylyl, myristyl, stearyl and other ethers of glucose and polyglucoside polymers, including mixed ethers such as capryl/caprylyl (C₈₋₁₀) glucoside, coco (C₈₋₁₆) glucoside, and lauryl (C₁₂₋₁₆) glucoside), long chain tertiary amine oxides, long chain tertiary phosphine oxides, long chain dialkyl sulfoxides, and mixtures of such materials.

In some embodiments, the nonionic surfactant system includes one or more of amine oxides, fatty acid amides, ethoxylated fatty alcohols, block copolymers of polyethylene glycol and polypropylene glycol, glycerol alkyl esters, polyoxyethylene glycol octylphenol ethers, sorbitan alkyl esters, polyoxyethylene glycol sorbitan alkyl esters, and mixtures thereof. Examples of amine oxides include, but are not limited to, laurylamidopropyl dimethylamine oxide, myristylamidopropyl dimethylamine oxide, and mixtures thereof. Examples of fatty acid amides include, but are not limited to, cocomonoethanolatnide, lauramide monoethanolamide, cocodiethanolamide, and mixtures thereof. In certain embodiments, the nonionic surfactant is a combination of an amine oxide and a fatty acid amide. In some such embodiments, the amine oxide is a mixture of laurylamidopropyl dimethylamine oxide and myristylamidopropyl dimethylamine oxide. In certain embodiments, the nonionic surfactant is a combination of lauryl/myristylamidopropyl dimethylamine oxide and cocomonoethanolamide. In certain embodiments, the nonionic surfactant system, i.e., the combined amount of nonionic surfactant in the composition, is present in an amount of about 0.1 to about 20 wt % of the composition. In other embodiments, the amount is about 0.1 to about 10, about 0.1 to about 6.0, about 0.5 to about 10, about 0.5 to about 5, or about 0.5 to about 3 wt %.

In some embodiments, the cationic surfactant and nonionic surfactant system are present in the composition in a weight ratio of cationic surfactant:nonionic surfactant system of greater than 0.8:1. Optionally, the ratio is at least 1:1. In some embodiments, the ratio of cationic surfactants to total nonionic surfactants is about 0.8:1 to about 1:0.8. In some embodiments, the ratio of cationic surfactants to total nonionic surfactants is about 0.8:1, about 0.81:1, about 0.82:1, about 0.83:1, about 0.84:1, about 0.85:1, about 0.86:1, about 0.87:1, about 0.88:1, about 0.89:1, about 0.9:1, about 0.91:1, about 0.92:1, about 0.93:1, about 0.94:1, about 0.95:1, about 0.96:1, about 0.97:1, about 0.98:1, about 0.99:1, about 1:1, about 1.01:1, about 1.02:1, about 1.03:1, about 1.04:1, about 1.05:1, about 1.06:1, about 1.07:1, about 1.08:1, about 1.09:1, about 1.1:1, about 1.11:1, about 1.12:1, about 1.13:1, about 1.14:1, about 1.15:1, about 1.16:1, about 1.17:1, about 1.18:1, about 1.19:1, or about 1.2:1.

In some embodiments, the personal care compositions of the disclosure include at least one structuring agent. The structuring agent may be added to compositions in the form of aqueous solutions, dispersions or emulsions. The structuring agent increases the viscosity of the composition. In various embodiments, the structuring agent is compatible with surfactant systems having both cationic and nonionic surfactants. In the invention, the structuring agent includes, or consists of, polyacrylate-1 crosspolymer. Polyacrylate-1 crosspolymer is sold under the tradename Carbopol© Aqua-CC from Lubrizol Advanced Materials, Inc.

In certain embodiments, the structuring agent is present in an amount of about 3.00, about 3.05, about 3.10, about 3.15, about 3.20, about 3.25, about 3.30, about 3.35, about 3.40, about 3.45, about 3.50, about 3.55, about 3.60, about 3.65, about 3.70, about 3.75, about 3.80, about 3.85, about 3.90, about 3.95, or about 4.00 wt % of the composition.

In some embodiments, the compositions of the disclosure can include one or more emollient components. Illustrative examples of such emollient components include mineral oils (e.g., paraffin oil, petroleum jelly oil), animal oils (e.g., fish oils and lanolin oil), vegetable oils (e.g., sweet almond oil, palm oil, avocado oil, olive oil, castor oil, cereal germ oil, canola oil, sunflower oil, soybean oil, and jojoba oil), triglycerides (e.g., caprylic/capric triglyceride), silicone oils (e.g., cyclomethicone), ester oils (e.g., butyl myristate, isopropyl myristate, cetyl myristate, isopropyl palmitate, isopropyl stearate, octyl stearate, isocearyl stearate), and organic fatty alcohols (e.g., oleic alcohol, linolenic alcohol, linoleic alcohol, isostearyl alcohol, octyl dodecanol).

Illustrative examples of emulsifying agents include ethoxylated carboxylic acids, ethoxylated glycerides, polyhydric alcohol ethers, and ethoxylated fatty alcohols.

In some embodiments, personal care compositions of the present disclosure further include one or more ingredients selected from coloring agents, fragrances, moisturizing agents, and amino acids.

In some embodiments, personal care compositions of the present disclosure include at least one viscosity modifier, useful for example to inhibit settling or separation of ingredients or to promote redispersibility upon agitation of a liquid composition. In some embodiments, the viscosity modifier is selected from a polymer and a hydrotrope. Optionally, the polymer comprises a block copolymer of propylene oxide and ethylene oxide, for example poloxamers. In some embodiments, the poloxamer comprises poloxamer 407, available under the trade name Pluronic® F127 from BASF Corporation. One or more viscosity modifiers are optionally present in a total amount of 0.01 wt % to 10 wt %, for example 0.1 wt % to 5 wt % or about 0.01 wt % to about 1 wt % of the composition.

In some embodiments, the disclosed compositions include a rheological profile capable of suspending beads, particles, or particulate matter. In some embodiments, the particles have shapes varying from spherical to irregular. The particles may be derived from inorganic or organic sources. Exemplary inorganic particles include carbonate salt, clay, silica, silicate, shale ash, perlite and quartz sand. Exemplary organic particles include polymeric beads like polypropylene, PVC, melamine, urea, polyacrylate and derivatives. The particles may also originate from natural sources. One type of suitable particles are natural particles such as nut shell particles and vegetable particles, and seed powders such as apricot seed powder. Exemplary natural particles include those derived from pistachio nut shell, walnut shell, almond shell and mixtures thereof. Other suitable natural particles include those derived from rice, corn cob, palm biomass, bamboo, kenaf, apple seeds, apricot stone, olive stone and mixtures thereof.

Further examples of beads include, but are not limited to, gelatin, celluloses, agar, gum arabic, alginates, carrageenan, waxes, polyethylenes, polyvinyl alcohol, poly(meth)acrylates, polystyrenes, polyurethanes, polyamides, polyepoxides, vinyl acetates, and polyvinyl pyrrolidones. Some preferred beads include but are not limited to those made from at least one of agar, alginate, or carrageenan, or a combination of two or more thereof. Some of the foregoing types of beads can be obtained from Lipo Technologies, Inc. under the tradename LIPOSPHERES or International Specialty Products under the tradename CAPTIVATES. These types of beads are porous and allow the bulk liquid that they are placed in to diffuse into the bead. This helps the beads become density matched to the composition to aid in suspension of the bead. Alternatively, materials can be encapsulated into beads to change their density to match the density of the bulk liquid.

The amount of beads in the composition can be any desired amount. In one embodiment, the amount of beads is 0.01% to 10% by weight of the composition, or 0.01 to 2% by weight.

In some embodiments, the beads are of any size that is viewable by a person. By viewable it is meant that the beads can be seen by a non-color blind person with an unaided eye at 20/20 or corrected to 20/20 with glasses or contact lenses at a distance of 30 cm from the composition under incandescent light, florescent light, or sunlight. In other embodiments, at least 50%, at least 60%, at least 70%, at least 80%, at least 90%, at least 95%, or at least 99% of the beads are viewable by a person. In one embodiment, the particle size is 100 to 2500 microns in a longest dimension of the bead. In another embodiment, the particle size is 250 to 2250 microns. In another embodiment, the particle size is 500 to 1500 microns. In another embodiment, the particle size is 700 to 1000 microns. In another embodiment, a combination of more than one particle size can be used. In another embodiment, there is a combination of five particle sizes.

Optional ingredients can be present in the personal care composition. Non-limiting examples include skin conditioning agents, moisturizing agents, fragrance, dyes and pigments, titanium dioxide, chelating agents such as EDTA, sunscreen active ingredients such as butyl methoxy benzoylmethane; antiaging compounds such as alpha hydroxy acids, beta hydroxy acids; preservatives such as hydantoins, imidazolines; polyols such as glycerol, sorbitol, propylene glycol and polyethylene glycols; particulate matter such as silica, talc, or calcium carbonate; antioxidants such as butylated hydroxytoluene (BHT); vitamins such as A, E, K and C; essential oils and extracts thereof such as rosewood and jojoba; particulate matter such as polyethylene beads, jojoba beads, lufa, or oat flour.

Some embodiments further include an antibacterial agent selected from a halogenated diphenyl ether (e.g. triclocarban), a magnolol derivative, an herbal extract or essential oil (e.g., rosemary extract, tea extract, thymol, menthol, eucalyptol, geraniol, carvacrol, citral, hinokitol, catechol, methyl salicylate, epigallocatechin gallate, epigallocatechin, gallic acid, miswak extract, sea-buckthorn extract), a bisguanide antiseptic (e.g., chlorhexidine, alexidine or octenidine), a quaternary ammonium compound (e.g., cetylpyridinium chloride (CPC), benzalkonium chloride, tetradecylpyridinium chloride (TPC), N-tetradecyl-4-ethylpyridinium chloride (TDEPC)), a phenolic antiseptic, hexetidine, octenidine, sanguinarine, povidone iodine, delmopinol, salifluor, metal ions (e.g., zinc salts, for example, zinc citrate, a stannous salt, a copper salt, an iron salt), sanguinarine, propolis and an oxygenating agent (e.g., hydrogen peroxide, buffered sodium peroxyborate or peroxycarbonate), phthalic acid or a salt thereof, monoperthalic acid or a salt or ester thereof, ascorbyl stearate, oleoyl sarcosine, alkyl sulfate, dioctyl sulfosuccinate, salicylanilide, domiphen bromide, delmopinol, octapinol or another piperidino derivative, a nicin preparation, a chlorite salt; and a combination of two or more thereof. In preferred embodiments, the antibacterial agent is selected from benzalkonium chloride, benzethonium chloride, and combinations thereof.

In some embodiments, the antibacterial agent is present in an amount of about 0.01% to about 5% of the total composition weight. In some embodiments, the benzalkonium chloride, benzethonium chloride, combination thereof is present in an amount of 0.01 to 1% of the total composition weight; for example about 0.1% to about 0.3% of the total composition weight.

In some embodiments, the personal care composition includes fragrance in an amount of about 0.001 wt % to about 2 wt % by weight of the composition.

In some embodiments, the personal care composition includes one or more pigments, such as chromium oxide green, in an amount of about 0.001 wt % to about 1 wt % by weight.

In some embodiments, the personal care composition includes silica, or silicon dioxide, incorporated at a level of from about 0.1 wt % to about 15 wt %, preferable from about 1 wt % to about 10 wt %, more preferably from about 3 wt % to about 7 wt %. Silica is available in a variety of forms, including but not limited to, crystalline, amorphous, fumed, precipitated, gel, and colloidal forms.

In some embodiments, the personal care composition includes inorganic salts, brighteners, perfumes, colorants, sequestering agents, opacifiers, chelating agents (e.g., EDTA), humectants (e.g., polyols, for example, glycerol), or any combination thereof.

In some embodiments, the personal care composition includes free fatty acids to provide enhanced skin feel benefits, such as softer or smoother feeling skin. Suitable free fatty acids include those derived from tallow, coconut oil, palm oil and palm kernel oil.

In a second exemplary embodiment, disclosed is a method (Method 1) of cleansing skin comprising the steps of providing a personal care cleansing composition as described above (e.g., any of Composition 1 and 1.1-1.27); and applying the composition to the skin to provide a cleansing effect.

The present disclosure provides additional exemplary embodiments, including:
1.1 Method 1, further comprising the step of agitating the composition on the skin sufficiently to create a volume of foam.
1.2 Any of Methods 1 or 1.1, wherein the volume of foam created is about 60% greater than a composition without polyacrylate-1 crosspolymer after 60 seconds of agitation.

In a third exemplary embodiment, disclosed is a method (Method 2) for preparing a personal care cleansing composition comprising combining an effective amount of polyacrylate-1 crosspolymer with a cationic surfactant and a nonionic surfactant.

The compositions herein can be prepared by procedures known in the art. In general, the various components of the composition are combined with water and mixed to uniformity. Premixes can be employed to pre-disperse or pre-dissolve components, and in particular powder components, and/or to add several components simultaneously.

Exemplary embodiments of the present disclosure will be illustrated by reference to the following examples, which are included to exemplify, but not to limit the scope of the present invention.

### EXAMPLES

### Example 1

Table 1 shows exemplary compositions according to the present disclosure arranged next to comparative compositions comprising little to no structuring agent.

**Table 1**

| **Ingredient** | **Comparative Comp. I** | **Comp. II** | **Comp. Ex. I** | **Comp. Ex. II** |
|---|---|---|---|---|
| | **Wt. %** | | | |
| Water | Q.S. | Q.S. | Q.S. | Q.S. |
| Polyacrylate-1 crosspolymer | 2.5 | 3.00 | 0.00 | 1.00 |
| Lauryl amidopropyl dimethyl amine oxide/myristyl amidopropyl dimethylamine oxide | 1.63 | 1.63 | 1.63 | 1.63 |
| Cocomonoethanolamide | 0.99 | 0.99 | 0.99 | 0.99 |
| EDTA-tetrasodium salt | 0.078 | 0.078 | 0.078 | 0.078 |
| Benzalkonium Chloride | 0.13 | 0.13 | 0.13 | 0.13 |
| Centrimonium Chloride | 2.75 | 2.75 | 2.75 | 2.75 |
| Glycerin | 2.00 | 2.00 | 2.00 | 2.00 |
| Fragrance | 0.40 | 0.40 | 0.40 | 0.40 |
| Colorant | 0.00 | 0.00 | 0.75 | 0.00 |
| Kathon CG | 0.0012 | 0.0012 | 0.0012 | 0.0012 |
| Beads | 1.90 | 1.90 | 0.00 | 1.90 |
| Citric acid | 0.80 | 0.80 | 0.80 | 0.80 |
| NaCl | 0.00 | 0.00 | 0.66 | 3.00 |
| Poloxamer | 0.30 | 0.90 | 0.00 | 0.00 |

Two exemplary compositions (Comparative Composition I and Composition II) are prepared in accordance with the formulas described in Table 1 (above). Comparative Composition I includes 2.5 wt % of polyacrylate-1 crosspolymer as a structurant; and Composition II includes 3.00 wt % of polyacrylate-1 crosspolymer as a structurant. Comparative Example I does not include polyacrylate-1 crosspolymer as a structurant; and Comparative Example II includes 1 wt % of polyacrylate-1 crosspolymer as a structurant.

As shown below, it was unexpectedly discovered that when relatively high concentrations of polyacrylate-1 crosspolymer, as in Comparative Composition I and Composition II, are added to a surfactant system having a cationic surfactant to nonionic surfactant ratio of about 0.8:1 at low pH, the composition exhibits enhanced rheological and foaming profiles.

### Example 2

A comprehensive rheological evaluation is conducted on each of Comparative Composition I, Composition II, Comparative Example I and Comparative Example II. The tests are carried out on a TA Series AR2000 or AR G2 stress controlled rheometer.

It is observed that when the structurant is present in the composition in an amount of 1 wt % or less, the compositions do not meet the structural parameter requirements to suspend particles.

**Table 2**

| | **Comp. I** | **Comp. II** | **Comp. Ex. I** | **Comp. Ex. II** |
|---|---|---|---|---|
| Ability to Suspend particles after 4 weeks storage | Yes | Yes | No | No |

In the tests conducted, the compositions are stored at 25°C for a period of four weeks. Immediately following the four-week storage period, the rheological parameters of the samples are analyzed. The results in Table 2 illustrate that in both Comparative Composition I and Composition II, the beads remained in suspension even following prolonged storage. In contrast, neither Comparative Example I nor Comparative Example II could maintain particles in suspension after four (4) weeks storage.

In order to suspend particles, the shear storage modulus (G') of the composition must be greater than the shear loss modulus (G") of the composition. That is, the ratio of G'/G" must be greater than 1:1. As shown in Table 3 (below), the ratios of G/G" of Compositions I and II as tested are unexpectedly high at 2.16 and 3.60, respectively. In contrast, Comparative Examples I and II have G'/G" ratios of 0.28 and 0.40, respectively, which equates with unsuitable structural performance, in the context of certain embodiments of the present invention.

**Table 3**

| **Comp. I** | **Comp. II** | **Comp. Ex. I** | **Comp. Ex. II** |
|---|---|---|---|
| 2.16 | 3.6 | 0.28 | 0.4 |

### Example 3

A foam profile evaluation is conducted on each of Comparative Composition I, Composition II, Comparative Example I, and Comparative Example II. The tests are carried out on a SITA Foam Tester R-2000. 250mL samples of each of the compositions are prepared and subjected to agitation for equal periods at a temperature of 40°C. It was found that the addition of a cationic structurant unexpectedly provides increased foam generation when subjected to agitation.

In the tests, Comparative Composition I, Composition II, Comparative Example I, and Comparative Example II are each subjected to agitation for a period of 80 seconds, and the volume of foam generated is recorded. Comparative Composition I and Composition II unexpectedly exhibit superior foaming over the entire test period in comparison to the comparative formulations (Comp. Ex. I and Comp. Ex. II). For example, after about 20 seconds of agitation, both Comparative Composition I and Composition II provide about 40% greater foam generation compared to the Comp. Ex. I. This difference grows to about 50% after 50 seconds of agitation. After 60 seconds of agitation, both exemplary compositions (Comparative **Comp.** I and Comp. II) demonstrate about 60% greater foam formation compared to both comparative formulations (Com. Ex. I and Comp. Ex. II).

### Example 4

An in-vitro antibacterial (AB) effiacy evaluation is conducted in accordance with the procedures of the European Standard NF EN 1040. The Short Interval Kill Time (SIKT) test is used to determine the in vitro short-term antimicrobial activity of compounds when tested in several exemplary compositions of the present invention. This test assesses the reduction of a microbial population of test organisms after exposure to a composition in-vitro. Test materials are mixed with bacterial inoculum for a selected time interval, after which the test system is neutralized and surviving bacteria enumerated. The SIKT contact time is 1 minute. Soap samples, or other viscous or solid samples, must be diluted. Bacterial reductions relative to water are used as the basis for expressing activity.

*Staphylococcus aureus* and *Escherichia coli* are selected as the test organisms. *E. coli* are part of the normal flora of the gut. *S. aureus* is frequently found on the skin where perspiration is present. *S. aureus* is a common cause of skin infections such as abscesses, respiratory infections such as sinusitis, and food poisoning. *S. aureus* is a gram-positive bacterium and *E. coli* is a gram-negative bacterium. Comparative Composition I and Comparative Example I are subjected to the SIKT analysis.

**Table 4**

| **Sample** | **Log Reduction** |
|---|---|
| Comparative Composition I | S. aureus = 7.05, E. coli =7 .31 |
| Comparative Example I | S. aureus = 7.05, E. coli =7 .31 |

The results for the SIKT test shown in Table 4 (above) demonstrate that Comparative Composition I showed equivalent efficacy with respect to both S. aureus and E. coli kill rate as Comparative Formulation 1. Thus, the presence of the structurant was found to have no negative effect on antibacterial efficacy.

## Claims

1. A personal care composition comprising:
a cationic surfactant;
a nonionic surfactant system comprising at least one surfactant chosen from an amine oxide and a fatty acid amide; and
a structuring agent comprising polyacrylate-1 crosspolymer in an amount of 3 wt % to 7 wt % by weight of the composition.

2. The personal care composition of claim 1, wherein the cationic surfactant comprises an alkyltrimethylammonium salt or cetyltrimethylammonium chloride.

3. The personal care composition of any preceding claim, further comprising a cationic antibacterial agent.

4. The personal care composition of any preceding claim, wherein the nonionic surfactant system comprises laurylamidopropyl dimethylamine oxide, myristylamidopropyl dimethylamine oxide, cocomonoethanolamide, or a combination thereof.

5. The personal care composition of any preceding claim, wherein the composition is a form selected from a liquid hand soap, a shampoo, a conditioner, a liquid facial soap, an antiperspirant, a deodorant, a body wash, a dermal lotion, a dermal cream, and a dermal conditioner.

6. The personal care composition of any preceding claim, wherein the cationic surfactant is present in an amount of from 0.1 wt % to 10 wt %, of the personal care composition, and wherein the nonionic surfactant system is present in an amount of from 0.1 wt % to 6.0 wt %, of the personal care composition.

7. The personal care composition of any preceding claim, wherein the composition has a shear storage modulus to shear loss modulus ratio of from 1:1 to 7:1.

8. The personal care composition of any preceding claim, wherein the weight ratio of cationic surfactant to nonionic surfactant system is from 0.8:1 to 1:0.8.

9. The personal care composition of any preceding claim wherein the pH of the composition is from 1 to 8.0, for example, from 3.0 to 7.0, from 4.0 to 6.0, from 5.0 to 5.4, or 5.0.

10. The personal care composition of any preceding claim further comprising one or more suspended particles; for example beads, for example alginate beads, agar beads, carrageenan beads, or a combination thereof.

11. The personal care composition of any preceding claim wherein the composition is substantially free of an anionic surfactant and wherein the cationic surfactant is a quaternary ammonium alkyl salt.

12. A method of cleansing skin comprising the steps of:
providing a personal care composition comprising a cationic surfactant, a nonionic surfactant system comprising at least one surfactant selected from an amine oxide and a fatty acid amide, and a structuring agent comprising polyacrylate-1 crosspolymer in an amount of 3 wt % to 7 wt % of the composition; and
applying the composition to the skin of a subject in need thereof.

13. The method of claim 12, further comprising the step of agitating the composition on the skin sufficiently to create a volume of foam.

14. The method of claim 12 or claim 13, wherein the composition has a shear storage modulus to shear loss modulus ratio from 2:1 to 4:1.

15. The method of any one of claims 12 to 14, wherein the cationic surfactant comprises an alkyltrimethylammonium salt, and wherein the nonionic surfactant system comprises laurylamidopropyl dimethylamine oxide, myristylamidopropyl dimethylamine oxide, cocomonoethanolamide, or a combination thereof, optionally wherein the composition is substantially free of an anionic surfactant and wherein the cationic surfactant is a quaternary ammonium alkyl salt.

## Patentansprüche

1. Körperpflegezusammensetzung, umfassend:
ein kationisches oberflächenaktives Mittel;
ein System aus nichtionischen oberflächenaktiven Mitteln, umfassend mindestens ein oberflächenaktives Mittel, ausgewählt aus einem Aminoxid und einem Fettsäureamid; und
ein Strukturiermittel, umfassend Polyacrylat-1-Crosspolymer in einer Menge von 3 Gew.-% bis 7 Gew.-%, bezogen auf das Gewicht der Zusammensetzung.

2. Körperpflegezusammensetzung nach Anspruch 1, wobei das kationische oberflächenaktive Mittel ein Alkyltrimethylammoniumsalz oder Cetyltrimethylammoniumchlorid umfasst.

3. Körperpflegezusammensetzung nach einem beliebigen vorhergehenden Anspruch, weiterhin umfassend ein kationisches antibakterielles Mittel.

4. Körperpflegezusammensetzung nach einem beliebigen vorhergehenden Anspruch, wobei das System aus nichtionischen oberflächenaktiven Mitteln Laurylamidopropyl-Dimethylaminoxid, Myristylamidopropyl-Dimethylaminoxid, Cocomonoethanolamid oder eine Kombination davon umfasst.

5. Körperpflegezusammensetzung nach einem beliebigen vorhergehenden Anspruch, wobei die Zusammensetzung eine Form aufweist, ausgewählt aus einer flüssigen Handseife, einem Shampoo, einem Conditioner, einer flüssigen Gesichtsseife, einem Antitranspirant, einem Deodorant, einem Körperwaschmittel, einer Hautlotion, einer Hautcreme und einem Hautconditioner.

6. Körperpflegezusammensetzung nach einem beliebigen vorhergehenden Anspruch, wobei das kationische oberflächenaktive Mittel in einer Menge von 0,1 Gew.-% bis 10 Gew.-%, bezogen auf die Körperpflegezusammensetzung, vorhanden ist und wobei das System aus nichtionischen oberflächenaktiven Mitteln in einer Menge von 0,1 Gew.-% bis 6,0 Gew.-%, bezogen auf die Körperpflegezusammensetzung, vorhanden ist.

7. Körperpflegezusammensetzung nach einem beliebigen vorhergehenden Anspruch, wobei die Zusammensetzung ein Verhältnis von Scherspeichermodul zu Scherverlustmodul von 1:1 bis 7:1 aufweist.

8. Körperpflegezusammensetzung nach einem beliebigen vorhergehenden Anspruch, wobei das Gewichtsverhältnis von kationischem oberflächenaktiven Mittel zu dem System aus nichtionischen oberflächenaktiven Mitteln 0,8:1 bis 1:0,8 beträgt.

9. Körperpflegezusammensetzung nach einem beliebigen vorhergehenden Anspruch, wobei der pH der Zusammensetzung 1 bis 8,0 beträgt, zum Beispiel 3,0 bis 7,0, 4,0 bis 6,0, 5,0 bis 5,4 oder 5,0.

10. Körperpflegezusammensetzung nach einem beliebigen vorhergehenden Anspruch, weiterhin umfassend ein oder mehrere suspendierte Partikel; zum Beispiel Kügelchen, zum Beispiel Alginatkügelchen, Agarkügelchen, Carrageenkügelchen oder eine Kombination davon.

11. Körperpflegezusammensetzung nach einem beliebigen vorhergehenden Anspruch, wobei die Zusammensetzung im Wesentlichen frei von einem anionischen oberflächenaktiven Mittel ist und wobei das kationische oberflächenaktive Mittel ein quaternäres Ammoniumalkylsalz ist.

12. Verfahren zur Hautreinigung, umfassend die Schritte:
Bereitstellen einer Körperpflegezusammensetzung, umfassend ein kationisches oberflächenaktives Mittel, ein System aus nichtionischen oberflächenaktiven Mitteln, umfassend mindestens ein oberflächenaktives Mittel, ausgewählt aus einem Aminoxid und einem Fettsäureamid, und ein Strukturiermittel, umfassend Polyacrylat-1-Crosspolymer in einer Menge von 3 Gew.-% bis 7 Gew.-%, bezogen auf die Zusammensetzung; und
Anwenden der Zusammensetzung an die Haut eines Subjekts das dessen bedarf.

13. Verfahren nach Anspruch 12, weiterhin umfassend den Schritt des Bewegens der Zusammensetzung auf der Haut, ausreichend, um ein Schaumvolumen zu erzeugen.

14. Verfahren nach Anspruch 12 oder Anspruch 13, wobei die Zusammensetzung ein Verhältnis von Scherspeichermodul zu Scherverlustmodul von 2:1 bis 4:1 aufweist.

15. Verfahren nach einem beliebigen der Ansprüche 12 bis 14, wobei das kationische oberflächenaktive Mittel ein Alkyltrimethylammoniumsalz umfasst, und wobei das System aus nichtionischen oberflächenaktiven Mitteln Laurylamidopropyldimethylaminoxid, Myristylamidopropyldimethylaminoxid, Cocomonoethanolamid oder eine Kombination davon umfasst, wobei die Zusammensetzung optional im Wesentlichen frei von einem anionischen oberflächenaktiven Mittel ist und wobei das kationische oberflächenaktive Mittel ein quaternäres Ammoniumalkylsalz ist.

## Revendications

1. Composition de soin personnel comprenant :
un tensioactif cationique ;
un système tensioactif non ionique comprenant au moins un tensioactif choisi parmi un oxyde d'amine et un amide d'acide gras ; et
un agent structurant comprenant un polymère réticulé polyacrylate-1 en une quantité de 3 % en poids à 7 % en poids de la composition.

2. Composition de soin personnel selon la revendication 1, dans laquelle le tensioactif cationique comprend un sel d'alkyltriméthylammonium ou du chlorure de cétyltriméthylammonium.

3. Composition de soin personnel selon l'une quelconque des revendications précédentes, comprenant en outre un agent antibactérien cationique.

4. Composition de soin personnel selon l'une quelconque des revendications précédentes, dans laquelle le système tensioactif non ionique comprend l'oxyde de laurylamidopropyl diméthylamine, l'oxyde de myristylamidopropyl diméthylamine, le cocomonoéthanolamide ou une combinaison de ceux-ci.

5. Composition de soin personnel selon l'une quelconque des revendications précédentes, dans laquelle la composition est une forme choisie parmi un savon liquide pour les mains, un shampooing, un après-shampoing, un savon liquide pour le visage, un anti-transpirant, un déodorant, un nettoyant pour le corps, une lotion dermique, une crème dermique et un revitalisant dermique.

6. Composition de soin personnel selon l'une quelconque des revendications précédentes, dans laquelle le tensioactif cationique est présent en une quantité de 0,1 % en poids à 10 % en poids, de la composition de soin personnel, et dans laquelle le système tensioactif non ionique est présent en une quantité de 0,1 % en poids à 6,0 % en poids de la composition de soin personnel.

7. Composition de soin personnel selon l'une quelconque des revendications précédentes, dans laquelle la composition a un rapport module de conservation sous cisaillement sur module de perte sous cisaillement de 1:1 à 7:1.

8. Composition de soin personnel selon l'une quelconque des revendications précédentes, dans laquelle le rapport pondéral du tensioactif cationique au système tensioactif non ionique est de 0,8:1 à 1:0,8.

9. Composition de soin personnel selon l'une quelconque des revendications précédentes, dans laquelle le pH de la composition est de 1 à 8,0, par exemple de 3,0 à 7,0, de 4,0 à 6,0, de 5,0 à 5,4 ou 5,0.

10. Composition de soin personnel selon l'une quelconque des revendications précédentes, comprenant en outre une ou plusieurs particules en suspension ; par exemple des billes, par exemple des billes d'alginate, des billes d'agar, des billes de carraghénane ou une combinaison de celles-ci.

11. Composition de soin personnel selon l'une quelconque des revendications précédentes, dans laquelle la composition est sensiblement exempte de tensioactif anionique et dans laquelle le tensioactif cationique est un sel d'alkyle d'ammonium quaternaire.

12. Procédé de nettoyage de la peau comprenant les étapes de :
fourniture d'une composition de soin personnel comprenant un tensioactif cationique, un système tensioactif non ionique comprenant au moins un tensioactif choisi parmi un oxyde d'amine et un amide d'acide gras, et un agent structurant comprenant un polymère réticulé polyacrylate-1 en une quantité de 3 % en poids à 7 % en poids de la composition ; et
application de la composition sur la peau d'un sujet en ayant besoin.

13. Procédé selon la revendication 12, comprenant en outre l'étape d'agitation de la composition sur la peau de manière suffisante pour créer un volume de mousse.

14. Procédé selon la revendication 12 ou la revendication 13, dans lequel la composition a un rapport module de conservation sous cisaillement sur module de perte sous cisaillement de 2:1 à 4:1.

15. Procédé selon l'une quelconque des revendications 12 à 14, dans lequel le tensioactif cationique comprend un sel d'alkyltriméthylammonium, et dans lequel le système tensioactif non ionique comprend l'oxyde de laurylamidopropyl diméthylamine, l'oxyde de myristylamidopropyl diméthylamine, le cocomonoéthanolamide, ou une combinaison de ceux-ci, éventuellement dans lequel la composition est sensiblement exempte de tensioactif anionique et dans lequel le tensioactif cationique est un sel d'alkyle d'ammonium quaternaire.
